# EUROPEAN PATENT SPECIFICATION

(11) **EP 3 440 190 B1**
(45) Date of publication and mention of the grant of the patent: **28.08.2024**
(21) Application number: 17716306.0
(22) Date of filing: 23.03.2017
(51) Int. Cl.: C12N 5/00

(54) **CELL CULTURE PROCESS**
ZELLKULTURVERFAHREN
PROCÉDÉ DE CULTURE CELLULAIRE

(30) Priority: 05.04.2016 US 201662318275 P
(43) Date of publication of application: 13.02.2019
(73) Proprietor: Pfizer Inc., New York, NY 10001-2192 (US)
(72) Inventor: FIGUEROA, Bruno, Cambridge, Massachusetts 02138 (US); LUAN, Yen-Tung, Chelmsford, Massachusetts 01824 (US); WANG, Wenge, Andover, Massachusetts 01810 (US)
(74) Representative: Pfizer
(86) International application number: PCT/IB2017/051692
(87) International publication number: WO 2017/175086

(56) References cited:
- WO-A1-2011/134920
- WO-A2-2006/050050
- US-A1- 2008 108 553
- US-B2- 7 294 484
- JAMES D. MICHAELS ET AL: "Interfacial properties of cell culture media with cell-protecting additives", BIOTECHNOLOGY AND BIOENGINEERING, vol. 47, no. 4, 20 August 1995 (1995-08-20), pages 420 - 430, XP055377405, ISSN: 0006-3592, DOI: 10.1002/bit.260470403

## Description

### Field of the invention

The invention relates to a cell culture medium comprising tyrosine at a concentration of at least 5 mM and polyvinylalcohol (PVA) between 0.5 and 5 g/L. The invention also relates to method of cell culture using a cell culture medium comprising tyrosine at a concentration of at least 5 mM and polyvinyl alcohol (PVA) between 0.5 and 5 g/L.

### Background

Proteins have become increasingly important as diagnostic and therapeutic agents. In most cases, proteins for commercial applications are produced in cell culture, from cells that have been engineered and/or selected to produce unusually high levels of a particular protein of interest. Optimization of cell culture conditions, including cell culture media, is important for successful commercial production of proteins. Like most amino acids in cell culture media, Tyrosine (Tyr) is needed for cell mass and antibody or protein production. A depletion of tyrosine in a production culture can lead to decreases in cell growth, viability and/or protein production. As a result of cell culture process improvements and need for high density and high titer process, cell culture media must comprises high concentration of amino acid to ensure cell growth and production of recombinant polypeptide. However, tyrosine has limited solubility at the desired concentration for maximal cell growth or protein production and tends to precipitate out of solution during storage at cold. One way to avoid the precipitation issue is to feed concentrated tyrosine stock separately. However, such approach adds operational complexity, especially for large scale manufacture process. In addition, concentrated tyrosine stock is high in pH and would cause pH changes during feed.

Therefore, there is a need for the development of improved cell culture media wherein solubility of tyrosine is increased for culturing mammalian cell at high density and for optimum production of proteins. US7294484 discloses media for the production of polypeptides (antibodies) by CHO cells with the media containing tyrosine and polyvinylalcohol (PVA).

### Summary of the invention

The invention is as defined in the claims.

In a first aspect of the invention there is provided a cell culture medium comprising tyrosine at a concentration of at least 5 mM and polyvinylalcohol (PVA), wherein the concentration of PVA is between 0.5 and 5 g/L.

In another aspect there is provided a method of cell culture comprising contacting mammalian cells with a cell culture medium as claimed.

In the claimed medium, the concentration of tyrosine is at least 5 mM. In some embodiments, the medium is a serum free and/or protein free medium.

In some embodiments, the cell culture is a fed batch culture and the cell culture medium of the invention is used as a base medium and/or a feed medium. In some embodiments, the maximum viable cell density during the cell culture is above 1 × 10⁶ cells/mL. In some embodiments, the cells express a recombinant protein.

### Detailed description of the invention

The present invention relates to methods and media for cell culture and for polypeptide production. The present invention relates to cell culture media comprising high concentration of tyrosine and polyvinyl alcohol (PVA).

In some embodiments, the concentration of tyrosine in the cell culture medium of the invention is at least 5mM, at least 6mM, at least 7mM, at least 8mM, at least 9mM or at least 10mM. The concentration of tyrosine in the cell culture medium of the invention at least 5mM. In some embodiments, the concentration of tyrosine in the cell culture medium of the invention is at least 6mM. In some embodiments, the concentration of tyrosine in the cell culture medium of the invention is comprised between 5mM and 20mM.

In some embodiments, the term tyrosine include any form of tyrosine suitable for being used as a nutrient in cell culture, preferably in animal cell culture, preferably in mammalian cell culture. In some embodiments, tyrosine is L-tyrosine. In some embodiments, tyrosine is tyrosine free base or a tyrosine salt. In some embodiments, tyrosine is a tyrosine salt selected from disodium salt or disodium salt dihydrate. In a preferred embodiment, tyrosine is L-tyrosine, L-tyrosine disodium or L-tyrosine disodium dihydrate.

PVA is a polymer of formula [-CH₂CH(OH)-]ₙ wherein n is an integer and represents the polymerization degree. In some embodiments, the PVA used in the cell culture medium of the invention is a polymer of formula [-CH₂CH(OH)-]ₙ wherein n is an integer comprised between 100 and 10000, 300 and 8000 or 500 and 5000. In some embodiments, the PVA used in the cell culture medium of the invention has a degree of hydrolysis of about 85% to about 89%. In some embodiments, the PVA used in the cell culture medium of the invention has a molecular weight between 10000 and 100000 Da. In some embodiments, the PVA used in the medium of the invention has a molecular weight between 10000 and 50000 Da, preferably between 13000 and 23000 Da. In some embodiments, the PVA used in the medium of the invention has a viscosity between 1 and 10 centipoise (cp), preferably between 3.0 and 4.5 cp. The viscosity of PVA is reported in terms of the viscosity of a 4% aqueous solution of the polyvinyl alcohol at a temperature of 20°C.

In some embodiments, the PVA used in the cell culture medium of the present invention is Selvol^{™} PVA 203 (Sekisui).

The concentration of PVA suitable for preventing precipitation of a cell culture medium comprising a specific concentration of tyrosine (of at least 3mM) can be determined easily by the skilled person, for example using a methodology similar to those disclosed in example 1. In some embodiments, the concentration of PVA in the cell culture medium of the invention is 1, 1.5, 2, 2.5, 3, 3.5, 4, 4.5, 5. In some embodiments, the concentration of PVA in the cell culture medium of the invention is 2.5 g/L.

The concentration of PVA in the cell culture medium of the invention is comprised between 0.5g/L and 5 g/L. In some embodiments, the concentration of PVA in the cell culture medium of the invention is comprised between 1g/L and 3g/L.

In some embodiments, the cell culture medium of the invention has a turbidity of less than 5NTU (Nephelometric Turbidity Unit) after 1, 2, 3 or 4 weeks storage at 4°C in the absence of light. In some embodiments, the cell culture medium of the invention has a turbidity of less than 5NTU after 2 weeks storage at 4°C in the absence of light. In some embodiments, the turbidity is measured as disclosed in the examples. In some embodiments, the turbidity is measured using a 2100P Turbidimeter (HACH).

In some embodiments, the cell culture medium of the invention comprises essentially no precipitate after 1, 2, 3 or 4 weeks storage at 4°C in the absence of light. In some embodiments, the cell culture medium of the invention comprises essentially no precipitate after 2 weeks storage at 4°C in the absence of light.

The terms "medium", "cell culture medium" and "culture medium" as used herein refer to a solution containing nutrients which nourish growing mammalian cells. Typically, such solutions provide essential and non-essential amino acids, vitamins, energy sources, lipids, and trace elements required by the cell for minimal growth and/or survival. In one embodiment, the medium may comprise Ala, Arg, Asn, Asp, Glu, Gly, His, Ile, Leu, Lys, Met, Phe, Pro, Ser, Thr, Trp, Tyr, Val and Cystine and/or Cys.

Such a solution may also contain supplementary components that enhance growth and/or survival above the minimal rate, including, but not limited to, hormones and/or other growth factors, particular ions (such as sodium, chloride, calcium, magnesium, and phosphate), buffers, vitamins, nucleosides or nucleotides, trace elements (inorganic compounds usually present at very low final concentrations), inorganic compounds present at high final concentrations (e.g., iron), amino acids, lipids, and/or glucose or other energy source. In some embodiments, a medium is advantageously formulated to a pH and salt concentration optimal for cell survival and proliferation. For example, the medium may be formulated to a pH between around 7.1 and 7.3 and a final osmolality between around 1000 and 1300mOsm.

In some embodiments, the medium is a feed medium that is added after the beginning of the cell culture. In one embodiment, the medium is a medium, preferably a feed medium comprising 4 to 10mM Ala, 30 to 60mM Arg, 50 to 90mM Asn, 10 to 30mM Asp, 2 to 40mM Glu, 2 to 15mM Gly, 8 to 20mM His, 25 to 32mM Ile, 35 to 60mM Leu, 28 to 60mM Lys, 9 to 25mM Met, 10 to 30mM Phe, 15 to 40mM Pro, 44 to 80mM Ser, 20 to 45mM Thr, 2 to 10mM Trp and 20 to 50mM Val.

A wide variety of mammalian growth media may be modified by adjusting tyrosine and PVA levels to be used in accordance with the present invention. For example, a medium according to the invention can be obtained by modifying the amount of tyrosine and PVA in known cell culture media such as media disclosed in WO06026445, EP2243827, WO02066603 or WO06050050.

In some embodiments, the medium is a chemically defined medium, wherein the components of the medium are both known and controlled. In some embodiments, the medium is a complex medium, in which not all components of the medium are known and/or controlled.

Chemically defined growth media for mammalian cell culture have been extensively developed and published over the last several decades. All components of defined media are well characterized, and so defined media do not contain complex additives such as serum or hydrolysates. Early media formulations were developed to permit cell growth and maintenance of viability with little or no concern for protein production. More recently, media formulations have been developed with the express purpose of supporting highly productive recombinant protein producing cell cultures. Such media are preferred for use in the method of the invention. Such media generally comprises high amounts of nutrients and in particular of amino acids to support the growth and/or the maintenance of cells at high density. If necessary, these media can be modified by the skilled person for use in the method of the invention. For example, the skilled person may add PVA and increase the amount of tyrosine in these media for their use as base media or feed media in a method as disclosed herein.

Not all components of complex media are well characterized, and so complex media may contain additives such as simple and/or complex carbon sources, simple and/or complex nitrogen sources, and serum, among other things. In some embodiments, complex media suitable for the present invention contains additives such as hydrolysates in addition to other components of defined medium as described herein.

In some embodiments, defined media typically includes roughly fifty chemical entities at known concentrations in water. Most of them also contain one or more well-characterized proteins such as insulin, IGF-1, transferrin or BSA, but others require no protein components and so are referred to as protein-free defined media. Typical chemical components of the media fall into five broad categories: amino acids, vitamins, inorganic salts, trace elements, and a miscellaneous category that defies neat categorization.

Cell culture medium may be optionally supplemented with supplementary components. The term "supplementary components" as used herein refers to components that enhance growth and/or survival above the minimal rate, including, but not limited to, hormones and/or other growth factors, particular ions (such as sodium, chloride, calcium, magnesium, and phosphate), buffers, vitamins, nucleosides or nucleotides, trace elements (inorganic compounds usually present at very low final concentrations), amino acids, lipids, and/or glucose or other energy source. In some embodiments, supplementary components may be added to the initial cell culture. In some embodiments, supplementary components may be added after the beginning of the cell culture.

Typically, trace elements refer to a variety of inorganic salts included at micromolar or lower levels. For example, commonly included trace elements are zinc, selenium, copper, and others. In some embodiments, iron (ferrous or ferric salts) can be included as a trace element in the initial cell culture medium at micromolar concentrations. Manganese is also frequently included among the trace elements as a divalent cation (MnCl₂ or MnSO₄) in a range of nanomolar to micromolar concentrations. Numerous less common trace elements are usually added at nanomolar concentrations.

In some embodiments, the medium of the invention is a medium suitable for supporting high viable cell density, such as for example 1 × 10⁶cells/mL, 5 × 10⁶cells/mL, 1 × 10⁷cells /mL, 5 × 10⁷ cells/mL, 1×10⁸ cells/mL or 5×10⁸ cells/mL, in a cell culture. In some embodiments, the cell culture is a mammalian cell fed-batch culture, preferably a CHO cells fed-batch culture.

The term "viable cell density" as used herein refers to the number of cells present in a given volume of medium. Viable cell density can be measured by any method known to the skilled person. Preferably, viable cell density is measured using an automated cell counter such as Bioprofile Flex^{®}. The term maximum cell density as used herein refers to the maximum cell density achieved during the cell culture. The term "cell viability" as used herein refers to the ability of cells in culture to survive under a given set of culture conditions or experimental variations. Those of ordinary skill in the art will appreciate that one of many methods for determining cell viability are encompassed in this invention. For example, one may use a dye (e.g., trypan blue) that does not pass through the membrane of a living cell, but can pass through the disrupted membrane of a dead or dying cell in order to determine cell viability.

### Cell culture methods

The terms "culture" and "cell culture" as used herein refer to a cell population that is suspended in a medium under conditions suitable to survival and/or growth of the cell population. As will be clear to those of ordinary skill in the art, in some embodiments, these terms as used herein refer to the combination comprising the cell population and the medium in which the population is suspended. In some embodiments, the cells of the cell culture are mammalian cells.

The medium of the invention may be used with any cell culture method that is amenable to the desired process (e.g., production of a recombinant protein (e.g., antibody)). As a non-limiting example, cells may be grown in batch or fed-batch cultures, where the culture is terminated after sufficient expression of the recombinant protein (e.g., antibody), after which the expressed protein (e.g., antibody) is harvested. Alternatively, as another non-limiting example, cells may be grown in batch-refeed, where the culture is not terminated and new nutrients and other components are periodically or continuously added to the culture, during which the expressed recombinant protein (e.g., antibody) is harvested periodically or continuously. Other suitable methods (e.g., spin-tube cultures) are known in the art and can be used to practice the present invention.

In some embodiments, a cell culture suitable for use with the medium of the invention is a fed-batch culture. The term "fed-batch culture" as used herein refers to a method of culturing cells in which additional components are provided to the culture at a time or times subsequent to the beginning of the culture process. Such provided components typically comprise nutritional components for the cells which have been depleted during the culturing process. A fed-batch culture is typically stopped at some point and the cells and/or components in the medium are harvested and optionally purified. In some embodiments, the fed-batch culture comprises a base medium supplemented with feed media. The medium of the invention can be used as a base medium and/or as a feed medium.

Cells may be grown in any convenient volume chosen by the practitioner. For example, cells may be grown in small scale reaction vessels ranging in volume from a few milliliters to several liters. Alternatively, cells may be grown in large scale commercial Bioreactors ranging in volume from approximately at least 500, 1000, 2500, 5000, 8000, 10,000, 12,000, 15000, 20000 or 25000 liters or more, or any volume in between. In some embodiments, the volume of the cell culture medium is at least 500L. In some embodiments, the volume of the cell culture medium is at least 5000L.

The temperature of a cell culture will be selected based primarily on the range of temperatures at which the cell culture remains viable and the range in which a high level of desired product (e.g., a recombinant protein) is produced. In general, most mammalian cells grow well and can produce desired products (e.g., recombinant proteins) within a range of about 25°C to 42°C, although methods taught by the present disclosure are not limited to these temperatures. Certain mammalian cells grow well and can produce desired products (e.g., recombinant proteins or antibodies) within the range of about 35°C to 40°C. In certain embodiments, a cell culture is grown at a temperature of 20, 21, 22, 23, 24, 25, 26, 27, 28, 29, 30, 31, 32, 33, 34, 35, 36, 37, 38, 39, 40, 41, 42, 43, 44 or 45°C at one or more times during the cell culture process. Those of ordinary skill in the art will be able to select appropriate temperature or temperatures in which to grow cells, depending on the particular needs of the cells and the particular production requirements of the practitioner. The cells may be grown for any amount of time, depending on the needs of the practitioner and the requirement of the cells themselves. In some embodiments, the cells are grown at a temperature between 35°C and 40°C. In some embodiments, the cells are grown at 37°C. In some embodiments, the cells are grown at 36.5°C.

In some embodiments, the cells may be grown during the initial growth phase (or growth phase) for a greater or lesser amount of time, depending on the needs of the practitioner and the requirement of the cells themselves. In some embodiments, the cells are grown for a period of time sufficient to achieve a predefined cell density. In some embodiments, the cells are grown for a period of time sufficient to achieve a predefined cell density of about 1 × 10⁶cells/mL, about 5 × 10⁶cells/mL, about 1 × 10⁷cells /mL, about 5 × 10⁷ cells/mL, about 1×10⁸ cells/mL or about 5×10⁸ cells/mL In some embodiments, the cells are grown for a period of time sufficient to achieve a cell density that is a given percentage of the maximal cell density that the cells would eventually reach if allowed to grow undisturbed. For example, the cells may be grown for a period of time sufficient to achieve a desired viable cell density of 1, 5, 10, 15, 20, 25, 30, 35, 40, 45, 50, 55, 60, 65, 70, 75, 80, 85, 90, 95 or 99 percent of maximal cell density. In some embodiments, the cells are grown until the cell density does not increase by more than 15%, 14%, 13%, 12%, 11%, 10%, 9%, 8%, 7%, 6%, 5%, 4%, 3%, 2% or 1% per day of culture. In some embodiments, the cells are grown until the cell density does not increase by more than 5% per day of culture.

In some embodiments the cells are allowed to grow for a defined period of time. For example, depending on the starting concentration of the cell culture, the temperature at which the cells are grown, and the intrinsic growth rate of the cells, the cells may be grown for 0, 1, 2, 3, 4, 5, 6, 7, 8, 9, 10, 11, 12, 13, 14, 15, 16, 17, 18, 19, 20 or more days, preferably for 4 to 10 days. In some cases, the cells may be allowed to grow for a month or more. The practitioner of the present invention will be able to choose the duration of the initial growth phase depending on protein production requirements and the needs of the cells themselves.

The cell culture may be agitated or shaken during the initial culture phase in order to increase oxygenation and dispersion of nutrients to the cells. In accordance with the present invention, one of ordinary skill in the art will understand that it can be beneficial to control or regulate certain internal conditions of the bioreactor during the initial growth phase, including but not limited to pH, temperature, oxygenation, etc.

At the end of the initial growth phase, at least one of the culture conditions may be shifted so that a second set of culture conditions is applied and a metabolic shift occurs in the culture. A metabolic shift can be accomplished by, e.g., a change in the temperature, pH, osmolality or chemical inductant level of the cell culture. In one non-limiting embodiment, the culture conditions are shifted by decreasing the temperature of the culture. However, as is known in the art, shifting temperature is not the only mechanism through which an appropriate metabolic shift can be achieved. For example, such a metabolic shift can also be achieved by shifting other culture conditions including, but not limited to, pH, osmolality, and sodium butyrate levels. The timing of the culture shift will be determined by the practitioner of the present invention, based on protein production requirements or the needs of the cells themselves.

When shifting the temperature of the culture, the temperature shift may be relatively gradual. For example, it may take several hours or days to complete the temperature change. Alternatively, the temperature shift may be relatively abrupt. For example, the temperature change may be complete in less than several hours. Given the appropriate production and control equipment, such as is standard in the commercial large-scale production of polypeptides or proteins, the temperature change may even be complete within less than an hour.

In some embodiments, once the conditions of the cell culture have been shifted as discussed above, the cell culture is maintained for a subsequent production phase under a second set of culture conditions conducive to the survival and viability of the cell culture and appropriate for expression of the desired polypeptide or protein at commercially adequate levels.

As discussed above, the culture may be shifted by shifting one or more of a number of culture conditions including, but not limited to, temperature, pH, osmolality, and sodium butyrate levels. In some embodiments, the temperature of the culture is shifted. According to this embodiment, during the subsequent production phase, the culture is maintained at a temperature or temperature range that is lower than the temperature or temperature range of the initial growth phase. As discussed above, multiple discrete temperature shifts may be employed to increase cell density or viability or to increase expression of the recombinant protein.

In some embodiments, the cells may be maintained in the subsequent production phase until a desired cell density or production titer is reached. In another embodiment of the present invention, the cells are allowed to grow for a defined period of time during the subsequent production phase. For example, depending on the concentration of the cell culture at the start of the subsequent growth phase, the temperature at which the cells are grown, and the intrinsic growth rate of the cells, the cells may be grown for 1, 2, 3, 4, 5, 6, 7, 8, 9, 10, 11, 12, 13, 14, 15, 16, 17, 18, 19, 20 or more days. In some cases, the cells may be allowed to grow for a month or more. The practitioner of the present invention will be able to choose the duration of the subsequent production phase depending on polypeptide or protein production requirements and the needs of the cells themselves.

The cell culture may be agitated or shaken during the subsequent production phase in order to increase oxygenation and dispersion of nutrients to the cells. In accordance with the present invention, one of ordinary skill in the art will understand that it can be beneficial to control or regulate certain internal conditions of the bioreactor during the subsequent growth phase, including but not limited to pH, temperature, oxygenation, etc.

In some embodiments, the cells express a recombinant protein and the cell culture method of the invention comprises a growth phase and a production phase.

### Cells

Any mammalian cell susceptible to cell culture may be utilized in accordance with the present invention. Non-limiting examples of mammalian cells that may be used in accordance with the present invention include BALB/c mouse myeloma line (NSO/I, ECACC No: 85110503); human retinoblasts (PER.C6, CruCell, Leiden, The Netherlands); monkey kidney CV1 line transformed by SV40 (COS-7, ATCC CRL 1651); human embryonic kidney line (293 or 293 cells subcloned for growth in suspension culture, Graham et al., J. Gen Virol., 36:59,1977); baby hamster kidney cells (BHK, ATCC CCL 10); Chinese hamster ovary cells +/-DHFR (CHO, Urlaub and Chasin, Proc. Natl. Acad. Sci. USA, 77:4216, 1980); mouse sertoli cells (TM4, Mather, Biol. Reprod., 23:243-251, 1980); monkey kidney cells (CV1 ATCC CCL 70); African green monkey kidney cells (VERO-76, ATCC CRL-1 587); human cervical carcinoma cells (HeLa, ATCC CCL 2); canine kidney cells (MOCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68, 1982); MRC 5 cells; FS4 cells; and a human hepatoma line (Hep G2). In some preferred embodiment, the cells are CHO cells. In some preferred embodiments, the cells are GS-CHO cells.

Additionally, any number of commercially and non-commercially available hybridoma cell lines may be utilized in accordance with the present invention. The term "hybridoma" as used herein refers to a cell or progeny of a cell resulting from fusion of an immortalized cell and an antibody-producing cell. Such a resulting hybridoma is an immortalized cell that produces antibodies. Individual cells used to create the hybridoma can be from any mammalian source, including, but not limited to, rat, pig, rabbit, sheep, pig, goat, and human. In some embodiments, a hybridoma is a trioma cell line, which results when progeny of heterohybrid myeloma fusions, which are the product of a fusion between human cells and a murine myeloma cell line, are subsequently fused with a plasma cell. In some embodiments, a hybridoma is any immortalized hybrid cell line that produces antibodies such as, for example, quadromas (See, e.g., Milstein et al., Nature, 537:3053, 1983). One skilled in the art will appreciate that hybridoma cell lines might have different nutrition requirements and/or might require different culture conditions for optimal growth, and will be able to modify conditions as needed.

### Expression of Proteins

As noted above, in many instances the cells will be selected or engineered to produce high levels of desired products (e.g., recombinant protein or antibody). Often, cells will be manipulated by the hand of man to produce high levels of recombinant protein, for example by introduction of a gene encoding the protein of interest and/or by introduction of genetic control elements that regulate expression of that gene (whether endogenous or introduced).

Certain proteins may have detrimental effects on cell growth, cell viability or some other characteristic of the cells that ultimately limits production of the protein of interest in some way. Even amongst a population of cells of one particular type engineered to express a specific protein, variability within the cellular population exists such that certain individual cells will grow better, produce more protein of interest, or produce a protein with higher activity levels (e.g., enzymatic activity). In certain embodiments, a cell line is empirically selected by the practitioner for robust growth under the particular conditions chosen for culturing the cells. In some embodiments, individual cells engineered to express a particular protein are chosen for large-scale production based on cell growth, final cell density, percent cell viability, titer of the expressed protein or any combination of these or any other conditions deemed important by the practitioner.

Any protein that is expressible in a host cell may be produced in accordance with the present teachings. The term "host cell" as used herein refers to a cell that is manipulated according to the present invention to produce a protein of interest as described herein. A protein may be expressed from a gene that is endogenous to the cell, or from a heterologous gene that is introduced into the cell. A protein may be one that occurs in nature, or may alternatively have a sequence that was engineered or selected by the hand of man.

Proteins that may desirably be expressed in accordance with the present invention will often be selected on the basis of an interesting or useful biological or chemical activity. For example, the present invention may be employed to express any pharmaceutically or commercially relevant enzyme, receptor, antibody, hormone, regulatory factor, antigen, binding agent, etc. In some embodiments, the protein expressed by cells in culture are selected from antibodies, or fragments thereof, nanobodies, single domain antibodies, glycoproteins, therapeutic proteins, growth factors, clotting factors, cytokines, fusion proteins, pharmaceutical drug substances, vaccines, enzymes, or Small Modular ImmunoPharmaceuticals^{™} (SMIPs). One of ordinary skill in the art will understand that any protein may be expressed in accordance with the present invention and will be able to select the particular protein to be produced based on his or her particular needs.

### Antibodies

Given the large number of antibodies currently in use or under investigation as pharmaceutical or other commercial agents, production of antibodies is of particular interest in accordance with the present invention. Antibodies are proteins that have the ability to specifically bind a particular antigen. Any antibody that can be expressed in a host cell may be produced in accordance with the present invention. In some embodiments, the antibody to be expressed is a monoclonal antibody.

In some embodiments, the monoclonal antibody is a chimeric antibody. A chimeric antibody contains amino acid fragments that are derived from more than one organism. Chimeric antibody molecules can include, for example, an antigen binding domain from an antibody of a mouse, rat, or other species, with human constant regions. A variety of approaches for making chimeric antibodies have been described. *See e.g.,* Morrison et al., Proc. Natl. Acad. Sci. U.S.A. 81, 6851 (1985); Takeda et al., Nature 314, 452 (1985), Cabilly et al., U.S. Patent No. 4,816,567; Boss et al., U.S. Patent No. 4,816,397; Tanaguchi et al., European Patent Publication EP171496; European Patent Publication 0173494, United Kingdom Patent GB 2177096B.

In some embodiments, the monoclonal antibody is a human antibody derived, e.g., through the use of ribosome-display or phage-display libraries (*see*, *e.g.*, Winter et al., U.S. Patent No. 6,291,159 and Kawasaki, U.S. Patent No. 5,658,754) or the use of xenographic species in which the native antibody genes are inactivated and functionally replaced with human antibody genes, while leaving intact the other components of the native immune system (*see, e.g.,* Kucherlapati et al., U.S. Patent No. 6,657,103).

In some embodiments, the monoclonal antibody is a humanized antibody. A humanized antibody is a chimeric antibody wherein the large majority of the amino acid residues are derived from human antibodies, thus minimizing any potential immune reaction when delivered to a human subject. In humanized antibodies, amino acid residues in the complementarity determining regions are replaced, at least in part, with residues from a nonhuman species that confer a desired antigen specificity or affinity. Such altered immunoglobulin molecules can be made by any of several techniques known in the art, (*e.g*., Teng et al., Proc. Natl. Acad. Sci. U.S.A., 80, 7308-7312 (1983); Kozbor et al., Immunology Today, 4, 7279 (1983); Olsson et al., Meth. Enzymol., 92, 3-16 (1982)), and are preferably made according to the teachings of PCT Publication WO92/06193 or EP 0239400, all of which are incorporated herein by reference). Humanized antibodies can be commercially produced by, for example, Scotgen Limited, 2 Holly Road, Twickenham, Middlesex, Great Britain. For further reference, see Jones et al., Nature 321:522-525 (1986); Riechmann et al., Nature 332:323-329 (1988); and Presta, Curr. Op. Struct. Biol. 2:593-596 (1992).

In some embodiments, the monoclonal, chimeric, or humanized antibodies described above may contain amino acid residues that do not naturally occur in any antibody in any species in nature. These foreign residues can be utilized, for example, to confer novel or modified specificity, affinity or effector function on the monoclonal, chimeric or humanized antibody. In some embodiments, the antibodies described above may be conjugated to drugs for systemic pharmacotherapy, such as toxins, low-molecular-weight cytotoxic drugs, biological response modifiers, and radionuclides (see e.g., Kunz et al., Calicheamicin derivative-carrier conjugates, US20040082764 A1).

In general, practitioners of the present invention will select a protein of interest, and will know its precise amino acid sequence. Any given protein that is to be expressed in accordance with the present invention may have its own particular characteristics and may influence the cell density or viability of the cultured cells, may be expressed at lower levels than another protein grown under identical culture conditions, and may have different biological activity depending on the exact culture conditions and steps performed. One of ordinary skill in the art will be able to appropriately modify the steps and compositions used to produce a particular protein according to the teachings of the present invention in order to optimize cell growth and the production and/or activity level of any given expressed protein.

### Introduction of Genes for the Expression of Proteins into Host Cells

Generally, a nucleic acid molecule introduced into the cell encodes the protein desired to be expressed according to the present invention. Alternatively, a nucleic acid molecule may encode a gene product that induces the expression of the desired protein by the cell. For example, introduced genetic material may encode a transcription factor that activates transcription of an endogenous or heterologous protein. Alternatively or additionally, an introduced nucleic acid molecule may increase the translation or stability of a protein expressed by the cell.

Methods suitable for introducing nucleic acids sufficient to achieve expression of a protein of interest into mammalian host cells are known in the art. See, for example, Gething et al., Nature, 293:620-625, 1981; Mantei et al., Nature, 281:40-46, 1979; Levinson et al. EP 117,060; and EP 117,058, For mammalian cells, common methods of introducing genetic material into mammalian cells include the calcium phosphate precipitation method of Graham and van der Erb (Virology, 52:456-457, 1978) or the lipofectamine^{™} (Gibco BRL) Method of Hawley-Nelson (Focus 15:73, 1993). General aspects of mammalian cell host system transformations have been described by Axel in U.S. Pat. No. 4,399,216 issued Aug. 16, 1983. For various techniques for introducing genetic material into mammalian cells, see Keown et al., Methods in Enzymology, 1989, Keown et al., Methods in Enzymology, 185:527-537, 1990, and Mansour et al., Nature, 336:348-352, 1988.

In some embodiments, a nucleic acid to be introduced is in the form of a naked nucleic acid molecule. For example, the nucleic acid molecule introduced into a cell may consist only of the nucleic acid encoding the protein and the necessary genetic control elements. Alternatively, a nucleic acid encoding the protein (including the necessary regulatory elements) may be contained within a plasmid vector. Non-limiting representative examples of suitable vectors for expression of proteins in mammalian cells include pCDNA1; pCD, see Okayama, et al. Mol. Cell Biol. 5:1136-1142, 1985; pMClneo Poly-A, see Thomas, et al. Cell 51:503-512, 1987; a baculovirus vector such as pAC 373 or pAC 610; CDM8 , see Seed, B. Nature 329:840, 1987; and pMT2PC, see Kaufman, et al. EMBO J. 6:187-195, 1987, each of which is incorporated herein by reference in its entirety. In some embodiments, a nucleic acid molecule to be introduced into a cell is contained within a viral vector. For example, a nucleic acid encoding the protein may be inserted into the viral genome (or a partial viral genome). Regulatory elements directing the expression of the protein may be included with the nucleic acid inserted into the viral genome (i.e., linked to the gene inserted into the viral genome) or can be provided by the viral genome itself.

Naked DNA can be introduced into cells by forming a precipitate containing the DNA and calcium phosphate. Alternatively, naked DNA can also be introduced into cells by forming a mixture of the DNA and DEAE-dextran and incubating the mixture with the cells or by incubating the cells and the DNA together in an appropriate buffer and subjecting the cells to a high-voltage electric pulse (e.g., by electroporation). A further method for introducing naked DNA cells is by mixing the DNA with a liposome suspension containing cationic lipids. The DNA/liposome complex is then incubated with cells. Naked DNA can also be directly injected into cells by, for example, microinjection.

Alternatively, naked DNA can also be introduced into cells by complexing the DNA to a cation, such as polylysine, which is coupled to a ligand for a cell-surface receptor (see for example Wu, G. and Wu, C.H. J. Biol. Chem. 263:14621, 1988; Wilson et al. J. Biol. Chem. 267:963-967, 1992; and U.S. Patent No. 5,166,320. Binding of the DNA-ligand complex to the receptor facilitates uptake of the DNA by receptor-mediated endocytosis.

Use of viral vectors containing particular nucleic acid sequences, e.g., a cDNA encoding a protein, is a common approach for introducing nucleic acid sequences into a cell. Infection of cells with a viral vector has the advantage that a large proportion of cells receive the nucleic acid, which can obviate the need for selection of cells which have received the nucleic acid. Additionally, molecules encoded within the viral vector, e.g., by a cDNA contained in the viral vector, are generally expressed efficiently in cells that have taken up viral vector nucleic acid.

Defective retroviruses are well characterized for use in gene transfer for gene therapy purposes (for a review see Miller, A.D. Blood 76:271, 1990). A recombinant retrovirus can be constructed having a nucleic acid encoding a protein of interest inserted into the retroviral genome. Additionally, portions of the retroviral genome can be removed to render the retrovirus replication defective. Such a replication defective retrovirus is then packaged into virions which can be used to infect a target cell through the use of a helper virus by standard techniques.

The genome of an adenovirus can be manipulated such that it encodes and expresses a protein of interest but is inactivated in terms of its ability to replicate in a normal lytic viral life cycle. See, for example, Berkner et al. BioTechniques 6:616, 1988; Rosenfeld et al. Science 252:431-434, 1991; and Rosenfeld et al. Cell 68:143-155, 1992. Suitable adenoviral vectors derived from the adenovirus strain Ad type 5 dl324 or other strains of adenovirus (e.g., Ad2, Ad3, Ad7 etc.) are known to those skilled in the art. Recombinant adenoviruses are advantageous in that they do not require dividing cells to be effective gene delivery vehicles and can be used to infect a wide variety of cell types, including airway epithelium (Rosenfeld *et al.,* 1992, cited supra), endothelial cells (Lemarchand et al., Proc. Natl. Acad. Sci. USA 89:6482-6486, 1992), hepatocytes (Herz and Gerard, Proc. Natl. Acad. Sci. USA 90:2812-2816, 1993) and muscle cells (Quantin et al., Proc. Natl. Acad. Sci. USA 89:2581-2584, 1992). Additionally, introduced adenoviral DNA (and foreign DNA contained therein) is not integrated into the genome of a host cell but remains episomal, thereby avoiding potential problems that can occur as a result of insertional mutagenesis in situations where introduced DNA becomes integrated into the host genome (e.g., retroviral DNA). Moreover, the carrying capacity of the adenoviral genome for foreign DNA is large (up to 8 kilobases) relative to other gene delivery vectors (Berkner *et al.* cited supra; Haj-Ahmand and Graham, J. Virol. 57:267, 1986). Most replication-defective adenoviral vectors currently in use are deleted for all or parts of the viral E1 and E3 genes but retain as much as 80% of the adenoviral genetic material.

Adeno-associated virus (AAV) is a naturally occurring defective virus that requires another virus, such as an adenovirus or a herpes virus, as a helper virus for efficient replication and a productive life cycle. (For a review see Muzyczka et al. Curr. Topics in Micro. and Immunol., 158:97-129, 1992). It is also one of the few viruses that may integrate its DNA into non-dividing cells, and exhibits a high frequency of stable integration (see for example Flotte et al., Am. J. Respir. Cell. Mol. Biol. 7:349-356, 1992; Samulski et al., J. Virol. 63:3822-3828, 1989; and McLaughlin et al., J. Virol. 62:1963-1973, 1989). Vectors containing as little as 300 base pairs of AAV can be packaged and can integrate. Space for exogenous DNA is limited to about 4.5 kb. An AAV vector such as that described in Tratschin et al. (Mol. Cell. Biol. 5:3251-3260, 1985) can be used to introduce DNA into cells. A variety of nucleic acids have been introduced into different cell types using AAV vectors (see for example Hermonat et al., Proc. Natl. Acad. Sci. USA 81:6466-6470, 1984; Tratschin et al., Mol. Cell. Biol. 4:2072-2081, 1985; Wondisford et al., Mol. Endocrinol. 2:32-39, 1988; Tratschin et al., J. Virol. 51:611-619, 1984; and Flotte et al., J. Biol. Chem. 268:3781-3790, 1993).

When the method used to introduce nucleic acid molecules into a population of cells results in modification of a large proportion of the cells and efficient expression of the protein by the cells, the modified population of cells may be used without further isolation or subcloning of individual cells within the population. That is, there may be sufficient production of the protein by the population of cells such that no further cell isolation is needed and the population can be immediately be used to seed a cell culture for the production of the protein. Alternatively, it may be desirable to isolate and expand a homogenous population of cells from a few cells or a single cell that efficiently produce(s) the protein.

Alternative to introducing a nucleic acid molecule into a cell that encodes a protein of interest, the introduced nucleic acid may encode another polypeptide or protein that induces or increases the level of expression of the protein produced endogenously by a cell. For example, a cell may be capable of expressing a particular protein but may fail to do so without additional treatment of the cell. Similarly, the cell may express insufficient amounts of the protein for the desired purpose. Thus, an agent that stimulates expression of the protein of interest can be used to induce or increase expression of that protein by the cell. For example, the introduced nucleic acid molecule may encode a transcription factor that activates or upregulates transcription of the protein of interest. Expression of such a transcription factor in turn leads to expression, or more robust expression of the protein of interest.

In certain embodiments, a nucleic acid that directs expression of the protein is stably introduced into the host cell. In certain embodiments, a nucleic acid that directs expression of the protein is transiently introduced into the host cell. One of ordinary skill in the art will be able to choose whether to stably or transiently introduce a nucleic acid into the cell based on his or her experimental needs.

A gene encoding a protein of interest may optionally be linked to one or more regulatory genetic control elements. In certain embodiments, a genetic control element directs constitutive expression of the protein. In certain embodiments, a genetic control element that provides inducible expression of a gene encoding the protein of interest can be used. The use of an inducible genetic control element (e.g., an inducible promoter) allows for modulation of the production of the protein in the cell. Non-limiting examples of potentially useful inducible genetic control elements for use in eukaryotic cells include hormone- regulated elements (e.g., see Mader, S. and White, J.H., Proc. Natl. Acad. Sci. USA 90:5603-5607, 1993), synthetic ligand-regulated elements (see, e.g. Spencer, D.M. et al., Science 262:1019-1024, 1993) and ionizing radiation-regulated elements (e.g., see Manome, Y. et al., Biochemistry 32:10607-10613, 1993; Datta, R. et al., Proc. Natl. Acad. Sci. USA 89:10149-10153, 1992). Additional cell-specific or other regulatory systems known in the art may be used in accordance with the invention.

One of ordinary skill in the art will be able to choose and, optionally, to appropriately modify the method of introducing genes that cause the cell to express the protein of interest in accordance with the teachings of the present invention.

### Isolation of the Expressed Protein

In general, it will typically be desirable to isolate and/or purify proteins expressed according to the present invention. In certain embodiments, the expressed protein is secreted into the medium and thus cells and other solids may be removed, as by centrifugation or filtering for example, as a first step in the purification process.

Alternatively, the expressed protein may be bound to the surface of the host cell. For example, the media may be removed and the host cells expressing the protein are lysed as a first step in the purification process. Lysis of mammalian host cells can be achieved by any number of means well known to those of ordinary skill in the art, including physical disruption by glass beads and exposure to high pH conditions.

The expressed protein may be isolated and purified by standard methods including, but not limited to, chromatography (e.g., ion exchange, affinity, size exclusion, and hydroxyapatite chromatography), gel filtration, centrifugation, or differential solubility, ethanol precipitation and/or by any other available technique for the purification of proteins (See, e.g., Scopes, Protein Purification Principles and Practice 2nd Edition, Springer-Verlag, New York, 1987; Higgins, S.J. and Hames, B.D. (eds.), Protein Expression : A Practical Approach, Oxford Univ Press, 1999; and Deutscher, M.P., Simon, M.I., Abelson, J.N. (eds.), Guide to Protein Purification : Methods in Enzymology (Methods in Enzymology Series, Vol. 182), Academic Press, 1997, each of which is incorporated herein by reference). For immunoaffinity chromatography in particular, the protein may be isolated by binding it to an affinity column comprising antibodies that were raised against that protein and were affixed to a stationary support. Alternatively, affinity tags such as an influenza coat sequence, poly-histidine, or glutathione-S-transferase can be attached to the protein by standard recombinant techniques to allow for easy purification by passage over the appropriate affinity column. Protease inhibitors such as phenyl methyl sulfonyl fluoride (PMSF), leupeptin, pepstatin or aprotinin may be added at any or all stages in order to reduce or eliminate degradation of the protein during the purification process. Protease inhibitors are particularly advantageous when cells must be lysed in order to isolate and purify the expressed protein.

One of ordinary skill in the art will appreciate that the exact purification technique will vary depending on the character of the protein to be purified, the character of the cells from which the protein is expressed, and/or the composition of the medium in which the cells were grown.

### Pharmaceutical Formulations

In certain embodiments of the invention, produced polypeptides or proteins will have pharmacologic activity and will be useful in the preparation of pharmaceuticals. Such produced polypeptide or protein may be administered to a subject or may first be formulated for delivery by any available route including, but not limited to parenteral (e.g., intravenous), intradermal, subcutaneous, oral, nasal, bronchial, ophthalmic, transdermal (topical), transmucosal, rectal, and vaginal routes. Pharmaceutical compositions typically include a purified polypeptide or protein expressed from a mammalian cell line, a delivery agent (i.e., a cationic polymer, peptide molecular transporter, surfactant, etc., as described above) in combination with a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use typically include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, NJ) or phosphate buffered saline (PBS). In all cases, the composition should be sterile and should be fluid to the extent that easy syringability exists. Preferred pharmaceutical formulations are stable under the conditions of manufacture and storage and must be preserved against the contaminating action of microorganisms such as bacteria and fungi. In general, the relevant carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, or sodium chloride in the composition. Prolonged absorption of the injectable compositions can be brought about by including in the composition an agent which delays absorption, for example, aluminum monostearate and gelatin.

Sterile injectable solutions can be prepared by incorporating the purified polypeptide or protein in the required amount in an appropriate solvent with one or a combination of ingredients enumerated above, as required, followed by filtered sterilization. Generally, dispersions are prepared by incorporating the purified polypeptide or protein expressed from a mammalian cell line into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the purified polypeptide or protein can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. Formulations for oral delivery may advantageously incorporate agents to improve stability within the gastrointestinal tract and/or to enhance absorption.

For administration by inhalation, the inventive compositions comprising a purified polypeptide or protein expressed from a mammalian cell line and a delivery agent are preferably delivered in the form of an aerosol spray from a pressured container or dispenser which contains a suitable propellant, *e.g*., a gas such as carbon dioxide, or a nebulizer. The present invention particularly contemplates delivery of the compositions using a nasal spray, inhaler, or other direct delivery to the upper and/or lower airway. Intranasal administration of DNA vaccines directed against influenza viruses has been shown to induce CD8 T cell responses, indicating that at least some cells in the respiratory tract can take up DNA when delivered by this route, and the delivery agents of the invention will enhance cellular uptake. According to certain embodiments of the invention the compositions comprising a purified polypeptide expressed from a mammalian cell line and a delivery agent are formulated as large porous particles for aerosol administration.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the purified polypeptide or protein and delivery agents are formulated into ointments, salves, gels, or creams as generally known in the art.

The compositions can also be prepared in the form of suppositories (*e.g*., with conventional suppository bases such as cocoa butter and other glycerides) or retention enemas for rectal delivery.

In some embodiments, the compositions are prepared with carriers that will protect the polypeptide or protein against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active polypeptide or protein calculated to produce the desired therapeutic effect in association with the required pharmaceutical carrier.

The polypeptide or protein expressed according to the present invention can be administered at various intervals and over different periods of time as required, e.g., one time per week for between about 1 to 10 weeks, between 2 to 8 weeks, between about 3 to 7 weeks, about 4, 5, or 6 weeks, etc. The skilled artisan will appreciate that certain factors can influence the dosage and timing required to effectively treat a subject, including but not limited to the severity of the disease or disorder, previous treatments, the general health and/or age of the subject, and other diseases present. Generally, treatment of a subject with a polypeptide or protein as described herein can include a single treatment or, in many cases, can include a series of treatments. It is furthermore understood that appropriate doses may depend upon the potency of the polypeptide or protein and may optionally be tailored to the particular recipient, for example, through administration of increasing doses until a preselected desired response is achieved. It is understood that the specific dose level for any particular animal subject may depend upon a variety of factors including the activity of the specific polypeptide or protein employed, the age, body weight, general health, gender, and diet of the subject, the time of administration, the route of administration, the rate of excretion, any drug combination, and the degree of expression or activity to be modulated.

The present disclosure that is not claimed includes the use of compositions for treatment of nonhuman animals. Accordingly, doses and methods of administration may be selected in accordance with known principles of veterinary pharmacology and medicine. Guidance may be found, for example, in Adams, R. (ed.), Veterinary Pharmacology and Therapeutics, 8th edition, Iowa State University Press; ISBN: 0813817439; 2001.

Pharmaceutical compositions can be included in a container, pack, or dispenser together with instructions for administration.

### Examples

### Example 1

Media comprising various concentration of L-Tyrosine (tyrosine 2Na2H₂O)), PVA (Sekisui Chemical Co, Selvol^{™} 203) and Pluronic F68 (Kolliphor P188, Sigma) and fixed concentrations of Ala, Arg, Asn, Asp, Cystine, Glu, Gly, His, Ile, Leu, Lys, Met, Phe, Pro, Ser, Thr, Trp, Tyr and Val, were prepared using feed medium A as starting material. Media were made according to the procedure described in Table 1 for medium comprising 5 g/L PVA and 3 or 12 mM Tyr, Table 2 for medium comprising no PVA, 5 g/L Pluronic F68 and 3 or 12 mM Tyr and Table 3 for medium comprising no PVA, no Pluronic F68 and 3 or 12 mM Tyr. The rest of the media were made by mixing the above 6 media to get the corresponding PVA, Pluronic F68 and Tyr concentrations. All media had a final osmolarity of 1000-1300 mOsm and a pH of 7.1-7.3. All media were sterile filtered through 0.2 µm filter unit and stored in 0.2 µm Nalgen filter bottles at cold (2-8°C) and protected from light.

**Table 1 - Medium with 5 g/L PVA, 3 mM or 12 mM Tyr**

| | | |
|---|---|---|
| Feed media A (powder) | 139.17 | g/L |
| PVA 60g/L | 83.3 | mL/L |
| Tyrosine 2Na 2H₂O (predissolve) | 0.785 or 3.14 | g/L |
| Addition of 10N NaOH to adjust pH to 9.0 +/-0.1 at 18-22°C | | |
| Acidic Cystine (400mM) | 11.7 | mL/L |
| pH adjust to 7.2+/-0.1 at18-22°C | | |

**Table 2 Media with 5 g/L Pluronic F68, 3 mM Tyr or 12 mM Tyr**

| | | |
|---|---|---|
| Feed media A (powder) | 139.17 | g/L |
| Pluronic F68 | 5 | g/L |
| Tyrosine 2Na 2H₂O (predissolve) | 0.785 or 3.14 | g/L |
| pH adjust to 9.0 +/-0.1 at18-22°C | | |
| Acidic Cystine (400mM) | 11.7 | mL/L |
| pH adjust to 7.2+/-0.1 at18-22°C | | |

**Table 3 Media with no PVA no Pluronic F68, 3 mM Tyr or 12 mM Tyr**

| | | |
|---|---|---|
| Feed media A (powder) | 139.17 | g/L |
| Tyrosine 2Na 2H2O (predissolve) | 0.785 or 3.14 | g/L |
| pH adjust to 9.0 +/-0.1 at18-22°C | | |
| Acidic Cystine (400mM) | 11.7 | mL/L |
| pH adjust to 7.2+/-0.1 at18-22°C | | |

Samples were taken at days indicated in Table 4 for turbidity measurement by a 2100P Turbidimeter (HACH) according to manufacturer instructions. D0 is the starting date for media storage for all conditions. Media with precipitations were not sampled for turbidity measurement for the rest of the sampling times after the precipitation, but continued to be stored at cold (2 to 8°C) and dark. Media with turbidity of 50 NTU and above is visible for precipitation.

**Table 4**

| | | | | D1 | | D4 | | D8 | | D15 | | D27 | |
|---|---|---|---|---|---|---|---|---|---|---|---|---|---|
| | PVA (g/L) | Plur. | Tyr (mM) | T | P | T | P | T | P | T | P | T | P |
| 1 | 0 | 0 | 3 | 1.67 | No | 1.73 | No | 1.78 | No | 1.5 | No | 1.55 | No |
| 2 | 0 | 0 | 6 | 1.76 | No | 1.66 | No | 1.63 | No | 1.54 | No | 6.99 | No |
| 3 | 0 | 0 | 9 | 1.79 | No | **122** | **Yes** | | | | | | |
| 4 | 0 | 0 | 12 | **79.8** | **Yes** | | | | | | | | |
| 5 | 2.5 | 0 | 3 | 1.77 | No | 1.74 | No | 1.77 | No | 1.71 | No | 9.47 | No |
| 6 | 2.5 | 0 | 6 | 1.67 | No | 2.41 | No | 1.67 | No | 1.66 | No | 1.82 | No |
| 7 | 2.5 | 0 | 9 | 1.62 | No | 1.64 | No | 1.63 | No | 1.45 | No | 1.92 | No |
| 8 | 2.5 | 0 | 12 | 1.57 | No | 1.59 | No | 1.69 | No | 1.35 | No | 7.94 | No |
| 9 | 0 | 2.5 | 3 | 1.76 | No | 1.77 | No | 2.62 | No | **98.1** | **Yes** | | |
| 10 | 0 | 2.5 | 6 | 1.74 | No | 2.11 | No | 2.19 | No | **152** | **Yes** | | |
| 11 | 0 | 2.5 | 9 | 7.14 | No | **95.7** | **Yes** | | | | | | |
| 12 | 0 | 2.5 | 12 | >Lim | **Yes** | | | | | | | | |
| 13 | 5 | 0 | 3 | 1.86 | No | 2.17 | No | 2.09 | No | 2.84 | No | 13.4 | No |
| 14 | 5 | 0 | 6 | 1.75 | No | 1.79 | No | 2.05 | No | 2.03 | No | 14.1 | No |
| 15 | 5 | 0 | 9 | 1.68 | No | 1.66 | No | 1.8 | No | 1.6 | No | 11.3 | No |
| 16 | 5 | 0 | 12 | 1.68 | No | 1.69 | No | 1.74 | No | 1.54 | No | 2.92 | No |
| 17 | 0 | 5 | 3 | 1.97 | No | 2.54 | No | **87.6** | **Yes** | | | | |
| 18 | 0 | 5 | 6 | 1.94 | No | 2.05 | No | **52.8** | **Yes** | | | | |
| 19 | 0 | 5 | 9 | 24.2 | No | **248** | **Yes** | | | | | | |
| 20 | 0 | 5 | 12 | >Lim | **Yes** | | | | | | | | |

| | | | | | | | | | | | | | |
|---|---|---|---|---|---|---|---|---|---|---|---|---|---|
| T: turbidity (NTU), P: precipitation, >Lim: above limit Turbidity below 5 NTU is normal. | | | | | | | | | | | | | |

Media with no PVA with a concentration of tyrosine higher than 6mM formed precipitates within 4 days (see samples 3 and 4). All media with Pluronic F68 have precipitated during storage time (4 weeks), with the higher tyrosine concentrations media precipitated earlier than that with lower tyrosine concentrations. In the presence of PVA, the turbidity remained low and no precipitate is observed, even after 27 days of storage.

## Claims

1. A cell culture medium comprising tyrosine at a concentration of at least 5 mM and polyvinylalcohol (PVA), wherein the concentration of PVA is between 0.5 and 5 g/L.

2. The cell culture medium according to claim 1 wherein the concentration of tyrosine is at least 10 mM.

3. The cell culture medium according to claim 1 wherein the concentration of tyrosine is between 5 and 20mM.

4. The cell culture medium according to any one of claims 1 to 3 wherein the concentration of PVA is at least 0.5g/L.

5. The cell culture medium according to any one of claims 1 to 3 wherein the concentration of PVA is 2.5 g/L.

6. The cell culture medium according to any one of claims 1 to 5 wherein said medium is protein free.

7. The cell culture medium according to any one of claims 1 to 6 wherein the turbidity is less than 5NTU after two weeks storage at 4°C in the absence of light.

8. The cell culture medium according to any one of claims 1 to 6 wherein essentially no precipitate is observed after two weeks storage at 4°C in the absence of light.

9. The cell culture medium according to any one of claims 1 to 8 wherein the medium comprises 4 to 10mM Ala, 30 to 60mM Arg, 50 to 90mM Asn, 10 to 30mM Asp, 2 to 40mM Glu, 2 to 15mM Gly, 8 to 20mM His, 25 to 32mM Ile, 35 to 60mM Leu, 28 to 60mM Lys, 9 to 25mM Met, 10 to 30mM Phe, 15 to 40mM Pro, 44 to 80mM Ser, 20 to 45mM Thr, 2 to 10mM Trp and 20 to 50mM Val.

10. A method of cell culture comprising contacting mammalian cells with a cell culture medium according to any one of claims 1 to 9.

11. The method of claim 10, wherein the mammalian cells are CHO cells.

12. The method of claim 10 or 11, wherein the cell culture is a fed batch culture.

13. The method of claim 12, wherein the fed batch culture comprises a base medium supplemented with feed media.

14. The method of claims 13, wherein only the base medium is a medium according to any one of claims 1 to 9.

15. The method of claim 13 wherein only the feed medium is a medium according to any one of claims 1 to 9.

16. The method of claim 13, wherein the base medium and the feed medium are media according to any one of claims 1 to 9.

17. The method of any one of claims 10 to 16 wherein the maximum viable cell density during the cell culture is above 1 × 10⁶ cells/mL.

18. The method of claim 17 wherein the maximum viable cell density is above 5 × 10⁶cells/mL, 1 × 10⁷cells /mL, 5 × 10⁷ cells/mL, 1×10⁸ cells/mL or 5×10⁸ cells/mL.

19. The method of any one of claims 10 to 18 wherein the volume of the cell culture medium is at least 500L.

20. The method of claim 19, wherein the volume of the cell culture medium is at least 5000L.

21. The method of any one of claims 10 to 20, wherein the cells express a recombinant protein.

22. The method of claim 21, wherein the recombinant protein is selected from the group consisting of antibodies or fragments thereof, nanobodies, single domain antibodies, Small Modular ImmunoPharmaceuticals (SMIPs), VHH antibodies, camelid antibodies, shark single domain polypeptides (IgNAR), single domain scaffolds (e.g., fibronectin scaffolds), single chain polypeptides comprising an N-terminal binding domain, an effector domain, and a C-terminal binding domain, growth factors, clotting factors, cytokines, fusion proteins, pharmaceutical drug substances, vaccines, enzymes and combinations thereof.

23. The method of claim 21 or 22, further comprising obtaining recombinant protein produced by the cells.

24. The method of claim 23, further comprising purifying the recombinant protein.

## Patentansprüche

1. Zellkulturmedium, umfassend Tyrosin in einer Konzentration von mindestens 5 mM und Polyvinylalkohol (PVA), wobei die PVA-Konzentration zwischen 0,5 und 5 g/L liegt.

2. Zellkulturmedium nach Anspruch 1, wobei die Tyrosinkonzentration mindestens 10 mM beträgt.

3. Zellkulturmedium nach Anspruch 1, wobei die Tyrosinkonzentration zwischen 5 und 20 mM liegt.

4. Zellkulturmedium nach einem der Ansprüche 1 bis 3, wobei die PVA-Konzentration mindestens 0,5 g/L beträgt.

5. Zellkulturmedium nach einem der Ansprüche 1 bis 3, wobei die PVA-Konzentration 2,5 g/L beträgt.

6. Zellkulturmedium nach einem der Ansprüche 1 bis 5, wobei das Medium proteinfrei ist.

7. Zellkulturmedium nach einem der Ansprüche 1 bis 6, wobei die Trübung nach zweiwöchiger Lagerung bei 4°C in Abwesenheit von Licht weniger als 5NTU beträgt.

8. Zellkulturmedium nach einem der Ansprüche 1 bis 6, wobei nach zweiwöchiger Lagerung bei 4°C in Abwesenheit von Licht im Wesentlichen kein Präzipitat beobachtet wird.

9. Zellkulturmedium nach einem der Ansprüche 1 bis 8, wobei das Medium 4 bis 10 mM Ala, 30 bis 60 mM Arg, 50 bis 90 mM Asn, 10 bis 30 mM Asp, 2 bis 40 mM Glu, 2 bis 15 mM Gly, 8 bis 20mM His, 25 bis 32mM Ile, 35 bis 60mM Leu, 28 bis 60mM Lys, 9 bis 25mM Met, 10 bis 30mM Phe, 15 bis 40mM Pro, 44 bis 80mM Ser, 20 bis 45mM Thr, 2 bis 10mM Trp und 20 bis 50mM Val umfasst.

10. Zellkulturverfahren, umfassend das Inkontaktbringen von Säugetierzellen mit einem Zellkulturmedium nach einem der Ansprüche 1 bis 9.

11. Verfahren nach Anspruch 10, wobei die Säugetierzellen CHO-Zellen sind.

12. Verfahren nach Anspruch 10 oder 11, wobei die Zellkultur eine gefütterte Batch-Kultur ist.

13. Verfahren nach Anspruch 12, wobei die gefütterte Batch-Kultur ein mit Feed-Medien ergänztes Basismedium umfasst.

14. Verfahren nach Anspruch 13, wobei nur das Basismedium ein Medium nach einem der Ansprüche 1 bis 9 ist.

15. Verfahren nach Anspruch 13, wobei nur das Feed-Medium ein Medium nach einem der Ansprüche 1 bis 9 ist.

16. Verfahren nach Anspruch 13, wobei das Basismedium und das Feed-Medium Medien nach einem der Ansprüche 1 bis 9 sind.

17. Verfahren nach einem der Ansprüche 10 bis 16, wobei die maximale lebensfähige Zelldichte während der Zellkultur über 1 × 10⁶ Zellen/ml liegt.

18. Verfahren nach Anspruch 17, wobei die maximale lebensfähige Zelldichte über 5 × 10⁶ Zellen/ml, 1 × 10⁷ Zellen/ml, 5 × 10⁷ Zellen/ml, 1 × 10⁸ Zellen/ml oder 5 × 10⁸ Zellen/ml liegt.

19. Verfahren nach einem der Ansprüche 10 bis 18, wobei das Volumen des Zellkulturmediums mindestens 500 L beträgt.

20. Verfahren nach Anspruch 19, wobei das Volumen des Zellkulturmediums mindestens 5000 L beträgt.

21. Verfahren nach einem der Ansprüche 10 bis 20, wobei die Zellen ein rekombinantes Protein exprimieren.

22. Verfahren nach Anspruch 21, wobei das rekombinante Protein ausgewählt ist aus der Gruppe bestehend aus Antikörpern oder Fragmenten davon, Nanokörpern, Single-Domain-Antikörpern, Small Modular ImmunoPharmaceuticals (SMIPs), VHH-Antikörpern, Camelid-Antikörpern, Hai-Single-Domain-Polypeptiden (IgNAR), Single-Domain-Scaffolds (z.B. Fibronectin-Gerüste), einkettigen Polypeptiden, die eine N-terminale Bindungsdomäne, eine Effektordomäne und eine C-terminale Bindungsdomäne umfassen, Wachstumsfaktoren, Gerinnungsfaktoren, Zytokinen, Fusionsproteinen, pharmazeutischen Wirkstoffen, Impfstoffen, Enzymen und Kombinationen davon.

23. Verfahren nach Anspruch 21 oder 22, das ferner die Gewinnung von rekombinantem Protein umfasst, das von den Zellen produziert wird.

24. Verfahren nach Anspruch 23, das ferner die Reinigung des rekombinanten Proteins umfasst.

## Revendications

1. Milieu de culture cellulaire comprenant de la tyrosine à une concentration d'au moins 5 mM et de l'alcool polyvinylique (PVA), dans lequel la concentration en PVA est entre 0,5 et 5 g/L.

2. Milieu de culture cellulaire selon la revendication 1 dans lequel la concentration en tyrosine est d'au moins 10 mM.

3. Milieu de culture cellulaire selon la revendication 1 dans lequel la concentration en tyrosine est entre 5 et 20 mM.

4. Milieu de culture cellulaire selon l'une quelconque des revendications 1 à 3 dans lequel la concentration en PVA est d'au moins 0,5 g/L.

5. Milieu de culture cellulaire selon l'une quelconque des revendications 1 à 3 dans lequel la concentration en PVA est de 2,5 g/L.

6. Milieu de culture cellulaire selon l'une quelconque des revendications 1 à 5 dans lequel ledit milieu est exempt de protéines.

7. Milieu de culture cellulaire selon l'une quelconque des revendications 1 à 6 dans lequel la turbidité est inférieure à 5 UTN après deux semaines de stockage à 4 °C en l'absence de lumière.

8. Milieu de culture cellulaire selon l'une quelconque des revendications 1 à 6 dans lequel sensiblement aucun précipité n'est observé après deux semaines de stockage à 4 °C en l'absence de lumière.

9. Milieu de culture cellulaire selon l'une quelconque des revendications 1 à 8 dans lequel le milieu comprend 4 à 10 mM d'Ala, 30 à 60 mM d'Arg, 50 à 90 mM d'Asn, 10 à 30 mM d'Asp, 2 à 40 mM de Glu, 2 à 15 mM de Gly, 8 à 20 mM d'His, 25 à 32 mM d'Ile, 35 à 60 mM de Leu, 28 à 60 mM de Lys, 9 à 25 mM de Met, 10 à 30 mM de Phe, 15 à 40 mM de Pro, 44 à 80 mM de Ser, 20 à 45 mM de Thr, 2 à 10 mM de Trp et 20 à 50 mM de Val.

10. Procédé de culture cellulaire comprenant la mise en contact de cellules de mammifère avec un milieu de culture cellulaire selon l'une quelconque des revendications 1 à 9.

11. Procédé selon la revendication 10, dans lequel les cellules de mammifère sont des cellules CHO.

12. Procédé selon la revendication 10 ou 11, dans lequel la culture cellulaire est une culture à écoulement discontinu.

13. Procédé selon la revendication 12, dans lequel la culture à écoulement discontinu comprend un milieu de base complété avec des milieux d'alimentation.

14. Procédé selon la revendication 13, dans lequel seul le milieu de base est un milieu selon l'une quelconque des revendications 1 à 9.

15. Procédé selon la revendication 13 dans lequel seul le milieu d'alimentation est un milieu selon l'une quelconque des revendications 1 à 9.

16. Procédé selon la revendication 13, dans lequel le milieu de base et le milieu d'alimentation sont des milieux selon l'une quelconque des revendications 1 à 9.

17. Procédé selon l'une quelconque des revendications 10 à 16 dans lequel la densité cellulaire viable maximale pendant la culture cellulaire est supérieure à 1 × 10⁶ cellules/ml.

18. Procédé selon la revendication 17 dans lequel la densité cellulaire viable maximale est supérieure à 5 × 10⁶ cellules/ml, 1 × 10⁷ cellules/ml, 5 × 10⁷ cellules/ml, 1 × 10⁸ cellules/ml ou 5 × 10⁸ cellules/ml.

19. Procédé selon l'une quelconque des revendications 10 à 18 dans lequel le volume du milieu de culture cellulaire est d'au moins 500 l.

20. Procédé selon la revendication 19, dans lequel le volume du milieu de culture cellulaire est d'au moins 5000 l.

21. Procédé selon l'une quelconque des revendications 10 à 20, dans lequel les cellules expriment une protéine recombinante.

22. Procédé selon la revendication 21, dans lequel la protéine recombinante est sélectionnée parmi le groupe constitué d'anticorps ou de fragments de ceux-ci, de nanocorps, d'anticorps à domaine unique, de produits immunopharmaceutiques modulaires de petite taille (SMIP), d'anticorps VHH, d'anticorps de camélidé, de polypeptides à domaine unique de requin (IgNAR), de squelettes à domaine unique (par ex., squelettes de fibronectine), de polypeptides à chaîne unique comprenant un domaine de liaison N-terminal, un domaine effecteur, et un domaine de liaison C-terminal, de facteurs de croissance, de facteurs de coagulation, de cytokines, de protéines de fusion, de substances médicamenteuses pharmaceutiques, de vaccins, d'enzymes et de combinaisons de ceux-ci.

23. Procédé selon la revendication 21 ou 22, comprenant en outre l'obtention d'une protéine recombinante produite par les cellules.

24. Procédé selon la revendication 23, comprenant en outre la purification de la protéine recombinante.
